(19) Europäisches Patentamt
European Patent Office
Office européen des brevets

(11) Veröffentlichungsnummer: **0 019 576**
**A1**

(12) # EUROPÄISCHE PATENTANMELDUNG

(21) Anmeldenummer: **80810107.5**

(22) Anmeldetag: **31.03.80**

(51) Int. Cl.³: **C 08 K 5/00**
**C 07 C 149/18**

(30) Priorität: **05.04.79 CH 3188/79**

(43) Veröffentlichungstag der Anmeldung:
**26.11.80 Patentblatt 80/24**

(84) Benannte Vertragsstaaten:
**BE DE FR GB**

(71) Anmelder: **CIBA-GEIGY AG**
**Patentabteilung Postfach**
**CH-4002 Basel(CH)**

(72) Erfinder: **Müller, Horst, Dr.**
**Reichenberger Strasse 12**
**D-6149 Fürth/Odenwald(DE)**

(72) Erfinder: **Wirth, Hermann O., Dr.**
**Lessingstrasse 24**
**D-6140 Bensheim 3(DE)**

(54) **Gemische aus beta-Ketocarbonsäureestern von Di- oder Polyolen mit Thioätherstruktur und metallhaltigen Stabilisatoren zur Stabilisierung von chlorhaltigen Thermoplasten.**

(57) Stabilisatorgemische für chlorhaltige Thermoplasten bestehend aus a) einem Thioäther der Formel I

$$(R\text{-}CO\text{-}\underset{\displaystyle R_1}{\underset{|}{CH}}\text{-}CO\text{-}O\text{-}Y\text{-}\underset{\displaystyle X}{\underset{|}{CH}})_2\text{-}S\text{-}(Z\text{-}S)_n$$

wie beispielsweise einer Verbindung der Formel
$(H_3C\text{-}CO\text{-}CH_2\text{-}COO\text{-}CH_2CH_2)_2\,S$
oder dem Reaktionsprodukt von Thiodiglycerin mit Acetessigsäuremethylester und

b) einem ein Metall der Grupper Zink, Calcium, Cadmium, Barium, Magnesiu, Zinn oder Antimon oder ferner auch Zink kombiniert mit mindestens einem der vorgenannten Metalle enthaltenden Stabilisator.

EP 0 019 576 A1

0019576



CIBA-GEIGY AG
Basel (Schweiz)

3-12294/+

Gemische aus β-Ketocarbonsäureestern von Di- oder Polyolen mit Thioätherstruktur und metallhaltigen Stabilisatoren zur Stabilisierung von chlorhaltigen Thermoplasten

Die vorliegende Erfindung betrifft neue Stabilisatorgemische bestehend aus β-Ketocarbonsäureestern von Di- oder Polyolen mit Thioätherstruktur und metallhaltigen Stabilisatoren zur Stabilisierung von chlorhaltigen Thermoplasten, sowie neue Costabilisatoren des obengenannten Thioäther-Typus.

Aus der DE-OS 1 569 407 ist ein ungiftiges Stabilisatorgemisch bestehend aus Diketoessigsäureestern und Metallsalzen von organischen Säuren zur Stabilisierung von Polyvinylchloridharzen, die insbesondere für Nahrungs- und Genussmittelverpackung zur Anwendung kommen sollen, bekannt. Es hat sich aber gezeigt, dass dieses Gemisch in der Praxis bezüglich der stabilisierenden Wirkung nicht immer ganz befriedigende Resultate zeigt.

Es ist nun gefunden worden, dass Gemische aus β-Ketocarbonsäureestern von Di- oder Polyolen mit Thioätherstruktur und metallhaltigen Stabilisatoren überraschenderweise eine ausgezeichnete, für die Praxis befriedigende Stabilisierung von chlorhaltigen

Thermoplasten gewährleisten, wobei ebenfalls toxikologisch bedenkenlose Produkte erhalten werden.

Demnach betrifft die vorliegende Erfindung Stabilisatorgemische bestehend aus

a) einem Thioäther der Formel I

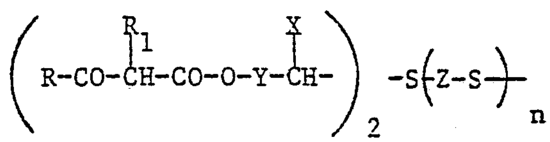

(I)

worin

n die Zahlen Null oder 1 bedeutet,

R $C_1$-$C_{18}$ Alkyl, $C_5$-$C_8$ Cycloalkyl, gegebenenfalls mit $C_1$-$C_4$ Alkyl substituiertes $C_6$-$C_{10}$ Aryl und

$R_1$ Wasserstoff oder $C_1$-$C_4$ Alkyl sind,

X als wiederholt vorkommendes Symbol gleich oder verschieden Wasserstoff oder eine Gruppe der Formel II

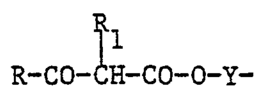

(II)

bedeutet,

Y als wiederholt vorkommendes Symbol gleich oder verschieden $C_1$-$C_6$ Alkylen oder eine Gruppe der Formel III

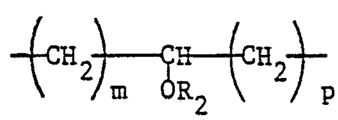

(III)

ist, wobei die $-(CH_2)_m$ Gruppe an das Sauerstoffatom gebunden ist und worin $R_2$ Wasserstoff oder eine Gruppe der Formel IV

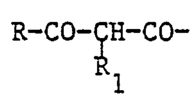

(IV)

bedeutet, m die Zahlen 1 bis 4 und p die Zahlen Null bis 3 sein können,

Z $C_1$-$C_6$ Alkylen oder eine Gruppe der Formel V

$$-CH_2-\underset{|\atop OR_2}{CH}-CH_2- \qquad (V)$$

ist, und

b) einem ein Metall aus der Gruppe Zink, Calcium, Cadmium, Barium, Magnesium, Zinn (bevorzugt Monoalkylzinn) oder Antimon oder ferner und bevorzugt auch Zink kombiniert mit mindestens einem der vorgenannten Metalle, insbesondere Calcium oder Barium enthaltenden Stabilisator.

Bedeuten etwaige Substituenten $C_1-C_4$ Alkyl, so kann es sich um Methyl, Aethyl, n-Propyl, Isopropyl, n-Butyl, sek.-Butyl oder tert.-Butyl, insbesondere jedoch um Methyl handeln.

R bedeutet als $C_1-C_{18}$ Alkyl z.B. Methyl, Aethyl, n-Propyl, Isopropyl, n-Butyl, sek.-Butyl, tert.-Butyl, n-Pentyl, n-Hexyl, n-Octyl, 1,1,3,3-Tetramethylbutyl, n-Nonyl, Decyl, Dodecyl, Tetradecyl, Hexadecyl oder Octadecyl. Bevorzugt ist R als Alkyl, $C_1-C_4$ Alkyl wie oben definiert und insbesondere Methyl.

Stellt R $C_5-C_8$ Cycloalkyl dar, so kann es sich um Cyclopentyl, Cycloheptyl, Cyclooctyl und insbesondere Cyclohexyl handeln.

Als $C_6-C_{10}$ Aryl bedeutet R z.B. Naphthyl oder insbesondere Phenyl.

Y und Z stellen als $C_1-C_6$ Alkylen z.B. Methylen, Aethylen, Propylen, Tetramethylen, Pentamethylen und Hexamethylen dar. Bevorzugt sind Methylen und Aethylen.

Als metallhaltige Stabilisatoren werden Ba/Cd-, Ba/Cd/Zn-, Organozinn, vorzugsweise Monoalkylzinn, und bevorzugt Ca/Zn- oder Ba/Zn-Stabilisatoren, insbesondere Ca- und Zn-Stearat verwendet.

Besonders interessant sind Stabilisatorgemische enthaltend als Komponente a) einen Thioäther der Formel I, worin

n die Zahlen Null oder 1 bedeutet,

R $C_1$-$C_4$ Alkyl, Cyclohexyl, gegebenenfalls mit $C_1$-$C_4$ Alkyl substituiertes Phenyl,

$R_1$ und X Wasserstoff sind

Y als wiederholt vorkommendes Symbol gleich oder verschieden, Methylen, Aethylen oder eine Gruppe der Formel III bedeutet, wobei die $-(CH_2)_m$ Gruppe an das Sauerstoffatom gebunden ist und worin $R_2$ Wasserstoff oder eine Gruppe der Formel IV bedeutet, worin R und $R_1$ die in dieser Bevorzugung bereits angegebene Bedeutung haben, m gleich 1 und p gleich Null oder 1 sind und

Z Methylen oder Aethylen bedeutet.

Bevorzugt sind Stabilisatorgemische enthaltend als Komponente a) einen Thioäther der Formel I, worin

n die Zahlen Null oder 1 bedeutet,

R Methyl und $R_1$ und X Wasserstoff sind,

Y als wiederholt vorkommendes Symbol gleich oder verschieden Methylen oder eine Gruppe der Formel VI

$$-CH_2-\underset{OR_2}{CH}- \qquad (VI)$$

bedeutet, wobei die $-CH_2$-Gruppe an das Sauerstoffatom gebunden ist und worin $R_2$ Wasserstoff oder eine Gruppe $CH_3-CO-CH_2-CO-$ ist und

Z Methylen bedeutet.

Einfache Verbindungen der Formel I, worin n gleich Null ist und Y Alkylen bedeutet, wie beispielsweise die Verbindung der Formel

$$\left(H_3C-CO-CH_2-CO-O-CH_2-CH_2-\right)_2S$$

sind aus der CH-PS 490 438, wo sie als Zusatzbeschleuniger für

Polyesterform- und Ueberzugsmassen beschrieben sind, bekannt.

Verbindungen der Formel I, worin n gleich 1 ist sowie diejenigen, worin Y eine Gruppe der Formel III bedeutet sind neu und stellen weitere Ausführungsformen der vorliegenden Erfindung dar.

Die Darstellung der erfindungsgemäss einzusetzenden Thioäthern der Formel I erfolgt sehr glatt durch Einwirkung von β-Ketocarbonsäureestern der Formel VII

$$R\text{-}CO\text{-}\underset{}{\overset{R_1}{\overset{|}{C}H}}\text{-}CO\text{-}O\text{-}CH_3 \qquad (VII)$$

auf Di- bzw. Polyole der Formel VIII

$$(HO\text{-}Y\text{-}\overset{X_1}{\overset{|}{C}H}\text{-})_2 \quad \text{-}S\text{-}(Z\text{-}S\text{-})_n \qquad (VIII)$$

worin $X_1$ Wasserstoff oder eine Gruppe -Y-OH bedeutet und alle anderen Symbole die oben angegebene Bedeutung haben.

Es ist vorteilhaft, diese Umesterung unter Zuhilfenahme einer Destillationskolonne vorzunehmen; das als Reaktionsprodukt gebildete Methanol lässt sich damit am besten abtrennen.

Wird totale Umesterung erstrebt, so ist es günstig, den Acetessigsäuremethylester in etwa der doppelten stöchiometrischen Menge einzusetzen. Unter stöchiometrischen Bedingungen wird nur eine partiell-statistische Umsetzung erreicht. Diese Produkte sind aber auch wertvolle Costabilisatoren. Die Temperatur für die Umesterung liegt im Bereich zwischen 120 und 160°C.

Ein zweiter Syntheseweg zu bevorzugten erfindungsgemässen Verbindungen beruht auf der Einwirkung von Diketen auf die hydroxylierten Thioäther der Formel VIII, wie z.B.:

2 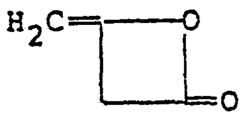 $+ HO-CH_2-CH_2-S-CH_2-S-CH_2-CH_2-OH$

$\longrightarrow (H_3C-CO-CH_2-CO-O-CH_2-CH_2-S)_2CH_2$

Als Reaktionsmedium wird vorteilhaft ein inertes Lösungsmittel (z.B. Toluol) benutzt. Die Mitverwendung eines Katalysators wie z.B. Pyridin ist von Vorteil.

Die hydroxylgruppenhaltigen Thioäther oder Thioformale, die als Ausgangsprodukte für die obengenannten Reaktionen dienen, sind an sich bekannt. Sollten einzelne von ihnen noch neu sein, so können sie in Analogie zu bekannten Reaktionen hergestellt werden, z.B. durch Umsetzung von $H_2S$ oder entsprechenden Mercaptanen mit Aethylenoxyd, Formaldehyd oder entsprechenden Epoxyverbindungen, wie beispielsweise

2 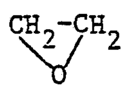 $+ H_2S \longrightarrow HO-CH_2-CH_2-S-CH_2-CH_2-OH$

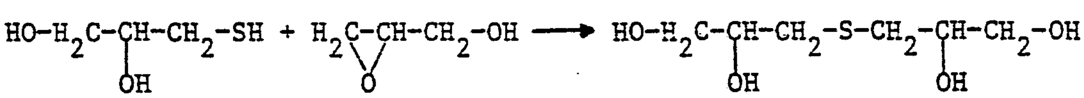

oder

$2\ HO-CH_2-CH_2-SH + H_2CO \longrightarrow HO-CH_2-CH_2-S-CH_2-S-CH_2-CH_2-OH$

Bei den β-Ketocarbonsäureestern der Formel VII handelt es sich um bekannte Verbindungen.

Für die weitere Umsetzung der Thioformale zu den erfindungsgemässen Verbindungen ist die Diketen-Methode besonders gut geeignet.

Das erfindungsgemässe synergistische Stabilisatorgemisch eignet sich hervorragend zum Schutz gegen den Abbau durch Wärmeeinwirkung von chlorhaltigen Thermoplasten.

Die Einzelbestandteile des Stabilisatorgemisches werden einzeln oder bereits vermischt den zu stabilisierenden Thermoplasten vor der Verarbeitung in üblichen Einrichtungen einverleibt, und zwar in jeweiligen Mengen von 0,05-1,5, bevorzugt 0,1-0,5 Gew.-%, bezogen auf die gesamte Zusammensetzung.

Als chlorhaltige Thermoplaste seien genannt: Polyvinylidenchlorid und bevorzugt Polymere aus oder auf der Grundlage von Vinylchlorid. Bevorzugt sind Suspensions- und Massepolymere und ausgewaschene, also emulgatorarme Emulsionspolymere. Beim Polyvinylchlorid kann es sich um weichmacherhaltiges oder um Hart-PVC handeln.

Als Comonomere für Thermoplaste auf der Grundlage von Vinylchlorid seien genannt: Vinilidenchlorid, Transdichloräthen, Aethylen, Propylen, Butylen, Maleinsäure, Acrylsäure, Fumarsäure oder Itaconsäure.

Vor, während oder nach der Zugabe des erfindungsgemässen Stabilisatorgemisches können je nach Verwendungszweck der Formmasse weitere Zusätze einverleibt werden.

Als weitere Additive, mit denen zusammen die erfindungsgemäss verwendbaren Stabilisatoren eingesetzt werden können, sind beispeilsweise zu nennen:

Antioxydantien, wie 2,6-Dialkylphenole, Derivate von alkylierten Hydrochinonen, hydroxylierte Thiodiphenyläther, Alkyliden-bisphenole, O-, N- und S-Benzylverbindungen, hydroxybenzylierte Malonester, Hydroxybenzyl-Aromaten, s-Triazinverbindungen, Amide der β-(3,5-Di-tert.-butyl-4-hydroxyphenyl)-propionsäure, Ester der β-(3,5-Di-tert.-butyl-4-hydroxyphenyl)-propionsäure, Ester der β-(5-tert.-Butyl-4-hydroxy-3-methylphenyl)-propionsäure, Ester der 3,5-Di-tert.-butyl-4-hydroxyphenylessigsäure, Acylaminophenole, Benzylphosphonate, Aminoarylderivate, UV-Absorber und Lichtschutzmittel, wie 2-(2'-Hydroxyphenyl)-benztriazole, 2,4-Bis-(2'-hydroxyphenyl)-6-alkyls-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phosphite, peroxidzerstörende Verbindungen, Polyamidstabilisatoren, basische Co-Stabilisatoren, Nukleierungsmittel oder sonstige Zusätze, wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in der DE-OS 2 427 853 auf Seiten 18 bis 24.

Das erfindungsgemässe Stabilisationsgemisch gewährleistet eine ausgezeichnete thermostabilisierende Wirkung, welche diejenige der Einzelkomponenten weit übersteigt.

Die nachfolgenden Beispiele dienen der näheren Erläuterung der Erfindung. Teile sind dabei Gewichtsteile und Prozente, Gewichtsprozente.

Herstellungsbeispiele

Beispiel 1: Umsetzung von Thiodiglycerin mit Acetessigsäuremethylester.

In einem Rundkolben mit Destillationsaufsatz werden 18,2 g (0,1 Mol) Thiodiglycerin mit 46,4g (0,4 Mol) Acetessigsäuremethylester auf ca. 150°C erhitzt. Der bei der Umesterung gebildete Methylalkohol wird in einer Vorlage aufgefangen. Nach Abtrennen des nicht umgesetzten Acetessigsäuremethylesters im Vakuum bei 60°C (13,1g) hinterbleiben 29,7 g einer gelben viskosen Flüssigkeit mit Brechungsindex $n_D^{20}$: 1,5043. Das erhaltene Produkt (Substanz 1) entspricht der Formel

$$H_3C\text{-}CO\text{-}CH_2\text{-}COO\text{-}CH_2\text{-}\underset{\displaystyle |\atop \displaystyle CH_3\text{-}CO\text{-}CH_2\text{-}COO}{CH}\text{-}CH_2\text{-}S\text{-}CH_2\text{-}\underset{\displaystyle |\atop \displaystyle OH}{CH}\text{-}CH_2OCO\text{-}CH_2\text{-}CO\text{-}CH_3$$

Wird das Thiodiglycerin mit überschüssigem Acetessigester umgesetzt, so lassen sich ohne Schwierigkeiten auch alle vier OH-Gruppen des Thiodiglycerins verestern.

Andererseits lassen sich mit unstöchiometrischen Mengen an Acetessigsäuremethylester auch Produkte mit höherem Gehalt an OH-Gruppen gewinnen.

Beispiel 2: Umsetzung von Thiodiglykol mit Acetessigsäuremethylester. In einem 1 l-Rundkolben mit aufgesetzter Destillationskolonne werden 122,2 g Bis-(2-hydroxyäthyl)-sulfid mit 465 g Acetessigsäuremethylester auf 130°C und langsam steigend auf 150°C erhitzt bis die zu erwartende Menge Methanol (66g) weitgehend abdestilliert ist.

Nach Entfernen der Kolonne wird unter vermindertem Druck der überschüssige Acetessigester abdestilliert und die letzten Reste mittels Rotationsverdampfer im Oelpumpenvakuum entfernt.

Es hinterbleibt ein schwach gelbgefärbtes Oel (Substanz 2), das ohne weitere Reinigung als Costabilisator eingesetzt werden kann. Es entspricht der Formel

$$(H_3C\text{-}CO\text{-}CH_2\text{-}CO\text{-}O\text{-}CH_2\text{-}CH_2\text{-})_2S$$

Brechungsindex: $n_D^{20}$ : 1,4889

Beispiel 3: Eine Mischung von 24,4 g (0,2 Mol) Thiodiäthylenglycol und 153 g (0,8 Mol) Benzoylessigsäureäthylester werden in einer Destillationsapparatur unter Rühren auf 160°C erwärmt, wobei ca.14 g (ber. 19,2 g) Aethanol abdestillieren. Das restliche Aethanol wird im Wasserstrahlvakuum bei einer Badtemperatur von 135°C entfernt. Anschliessend wird der überschüssige Benzoylessigsäureäthylester im Oelpumpenvakuum abdestilliert. Das zurückbleibende Produkt (74,4 g ≅ 90% d.Th.) enthält mehr als 95% Thiodiäthylenglycol-bis-benzoylacetat (Substanz 3).
Brechungsindex: $n_D^{20}$ : 1,5785.

Applikationsbeispiele

A. Eine Trockenmischung bestehend aus 100 Teilen S-PVC (K-Wert 58), 2 Teilen epoxidiertem Sojabohnenöl, 0,9 Teilen Ca-Stearat, 0,6 Teilen Zn-Stearat und der in der nachstehenden Tabelle in % (bezogen auf die Gesamtzusammensetzung) angegebenen Menge an Kostabilisator mit Thioätherstruktur wird auf einem Mischwalzwerk 5 Minuten bei 180°C gewalzt. Vom gebildeten Walzfell werden Testfolienstücke von 0,3 mm Dicke entnommen.

Die Folienproben werden in einem Ofen bei 180°C thermisch belastet und im zeitlichen Abstand von 5 Minuten an einer Probe der Yellowness-Index (YI) nach ASTMD 1925-70 bestimmt.

Die Ergebnisse sind in der nachstehenden Tabelle 1 zusammengefasst.

Tabelle 1

| Test Nr. | Kostabilisator | % | Walzfolie | Yellowness-Index nach 5 Min. | 10 Min. | 15. Min | 20 Min. | 25 Min. | 30 Min. |
|---|---|---|---|---|---|---|---|---|---|
| 1 | - | - | 37,3 | 54,0 | 55,6 | 51,3 | 48,3 | 50,7 | schwarz |
| 2 | Substanz 1 | 0,2 | 15,7 | 24,0 | 31,2 | 33,4 | 44,3 | 43,2 | 80,9 |
| 3 | " 1 | 0,4 | 8,8 | 13,7 | 18,1 | 17,5 | 25,9 | 26,1 | 53,9 |
| 4 | " 2 | 0,2 | 9,3 | 14,2 | 17,4 | 18,9 | 26,9 | 30,8 | schwarz |
| 5 | " 2 | 0,4 | 7,8 | 13,5 | 14,3 | 16,3 | 29,9 | 44,6 | " |

Die Walzfelle wurden ferner bei 180°C und 200 atü während 1 Minute zu 1 mm starken Platten verpresst und deren Yellownessindex gemessen. Die Messergebnisse sind folgender Tabelle 2 zu entnehmen:

Tabelle 2

| Test Nr. | Kostabilisator | % | Yellowness-Index |
|---|---|---|---|
| 1 | - | - | 132,3 |
| 2 | Substanz 1 | 0,2 | 73,3 |
| 3 | " 1 | 0,4 | 43,3 |
| 4 | " 2 | 0,2 | 47,8 |
| 5 | " 2 | 0,4 | 32,3 |

B. Eine zur Herstellung von transparenten Flaschen geeignete Rezeptur hatte folgenden Aufbau:

| | |
|---|---|
| Suspensions- PVC (K Wert 58) | 100 Tl. |
| Epoxidiertes Sojaöl | 3 Tl. |
| Calziumstearat | 0,2 Tl. |
| Zinkstearat | 0,2 Tl. |
| Gleitmittel | 1,2 Tl. |
| Thiodiäthylenglykol-bis-benzoylacetat | 0,5 Tl. |

Das in einem Fluidmischer hergestellte Dry-Blend wurde bei 190°C gewalzt und dem Walzfell im zeitlichen Abstand von 5' Proben entnommen, deren Verfärbung gemessen wurde (Yellownessindex). Die Resultate sind folgender Tabelle zu entnehmen:

| Kostabilisator | Yellowness-Index nach | | | | | |
|---|---|---|---|---|---|---|
| | 5 Min. | 10 Min. | 15. Min | 20 Min. | 25 Min. | 30 Min. |
| Substanz 3 | 5,8 | 8,0 | 15,0 | 25,3 | 53,4 | 77,0 |

Patentansprüche

1. Stabilisatorgemische bestehend aus

a) einem Thioäther der Formel I

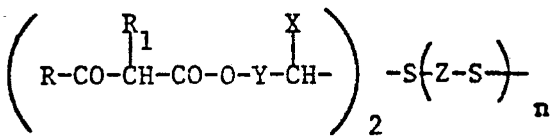

(I)

worin

n die Zahlen Null oder 1 bedeutet,

R $C_1$-$C_{18}$ Alkyl, $C_5$-$C_8$ Cycloalkyl, gegebenenfalls mit $C_1$-$C_4$ Alkyl substituiertes $C_6$-$C_{10}$ Aryl und

$R_1$ Wasserstoff oder $C_1$-$C_4$ Alkyl sind,

X als wiederholt vorkommendes Symbol gleich oder verschieden Wasserstoff oder eine Gruppe der Formel II

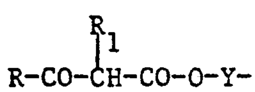

(II)

bedeutet,

Y als wiederholt vorkommendes Symbol gleich oder verschieden $C_1$-$C_6$ Alkylen oder eine Gruppe der Formel III

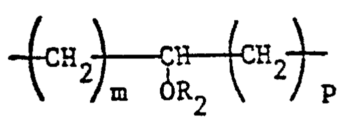

(III)

ist, wobei die $-(CH_2)_m-$ Gruppe and das Sauerstoffatom gebunden ist und worin $R_2$ Wasserstoff oder eine Gruppe der Formel IV

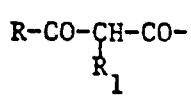

(IV)

bedeutet, m die Zahlen 1 bis 4 und p die Zahlen Null bis 3 sein können,

Z $C_1$-$C_6$ Alkylen oder eine Gruppe der Formel V

$$-CH_2-\underset{OR_2}{CH}-CH_2- \qquad (V)$$

ist, und

b) einem ein Metall aus der Gruppe Zink, Calcium, Cadmium, Barium, Magnesium, Zinn oder Antimon oder ferner auch Zink kombiniert mit mindestens einem der vorgenannten Metalle enthaltenden Stabilisator.

2. Stabilisatorgemische gemäss Anspruch 1, dadurch gekennzeichnet, dass sie als Komponente a) einen Thioäther der Formel I enthalten, worin

n die Zahlen Null oder 1 bedeutet,

R $C_1$-$C_4$ Alkyl, Cyclohexyl, gegebenenfalls mit $C_1$-$C_4$ Alkyl substituiertes Phenyl,

$R_1$ und X Wasserstoff sind,

Y als wiederholt vorkommendes Symbol gleich oder verschieden, Methylen, Aethylen oder eine Gruppe der Formel III bedeutet, wobei die $-(CH_2)_m$ Gruppe an das Sauerstoffatom gebunden ist und worin $R_2$ Wasserstoff oder eine Gruppe der Formel IV bedeutet, worin R und $R_1$ die in diesem Anspruch bereits angegebene Bedeutung haben, m gleich 1 und p gleich Null oder 1 sind, und

Z Methylen oder Aethylen bedeutet.

3. Stabilisatorgemische gemäss Anspruch 1, dadurch gekennzeichnet, dass sie als Komponente a) einen Thioäther der Formel I enthalten, worin

n die Zahlen Null oder 1 bedeutet,

R Methyl und $R_1$ und X Wasserstoff sind,

Y als wiederholt vorkommendes Symbol gleich oder verschieden Methylen oder eine Gruppe der Formel VI

$$-CH_2-\underset{OR_2}{CH}- \qquad (VI)$$

bedeutet, wobei die $-CH_2$-Gruppe an das Sauerstoffatom gebunden ist und worin $R_2$ Wasserstoff oder eine Gruppe $CH_3-CO-CH_2-CO-$ ist und

Z Methylen bedeutet.

4. Stabilisatorgemische gemäss Anspruch 1, dadurch gekennzeichnet, dass sie als Komponente b) einen Zink kombiniert mit Calcium oder Barium enthaltenden Stabilisator beinhalten.

5. Stabilisatorgemische gemäss Anspruch 1, dadurch gekennzeichnet, dass sie als Komponente b) einen Ca- und Zn-Stearatgemisch enthalten.

6. Stabilisierte Zusammmensetzung enthaltend ein chlorhaltiges thermoplastisches Polymer und als Stabilisator ein Stabilisatorgemisch gemäss Anspruch I, wobei die Komponenten a) und b) in jeweiligen Mengen von 0,05 - 1,5 Gew.%, bezogen auf die gesamte Zusammensetzung enthalten sind.

7. Stabilisierte Zusammensetzung gemäss Anspruch 6, dadurch gekennzeichnet, dass sie zusätzlich einen Lichtstabilisator enthält.

8. Stabilisierte Zusammensetzung gemäss den Ansprüchen 6 und 7, dadurch gekennzeichnet, dass der chlorhaltige Thermoplast ein Polymer aus oder auf der Grundlage von Vinylchlorid ist.

9. Verbindungen der Formel I, worin n gleich 1 ist und alle übrigen Symbole die in Anspruch 1 angegebene Bedeutung haben.

10. Verbindungen der Formel I, worin Y eine Gruppe der Formel III bedeutet und alle übrigen Symbole die in Anspruch 1 angegebene Bedeutung haben.

0019576

Europäisches Patentamt

# EUROPÄISCHER RECHERCHENBERICHT

Nummer der Anmeldung
EP 80 81 0107

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - A - 1 817 109 (DEUTSCHE ADVANCE)<br>* Anspruch; Seite 3, letzter Absatz; Seite 4, Absätze 1-3; Seite 7, Tabelle I; Beispiel 4 *<br>-- | 1 | C 08 K 5/00<br>C 07 C 149/18 |
| A | CH - A - 406 193 (MONTECATINI) | | |
| A | CH - A - 482 748 (STAUFFER CHEMICAL)<br>---- | | |

RECHERCHIERTE SACHGEBIETE (Int. Cl. 3)

C 08 K 5/00
5/36
C 07 C 149/18
C 08 L 27/06

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 17-09-1980 | LENSEN |